# EUROPEAN PATENT APPLICATION

(11) **EP 4 278 993 A1**
(43) Date of publication of application: **22.11.2023**
(21) Application number: 23172869.2
(22) Date of filing: 11.05.2023
(51) Int. Cl.: A61B 17/32

(54) **SURGICAL INSTRUMENT SHAFT ASSEMBLY WITH CONCENTRIC SEGMENTED FLEXIBLE TUBES**

(30) Priority: 18.05.2022 US 202217747690
(71) Applicant: Lenkbar, LLC, Naples, FL 34104 (US)
(72) Inventor: PAPENFUSS, Erik H., Naples, FL, 34104 (US)
(74) Representative: Kransell & Wennborg KB

(57) **Abstract**

A surgical instrument shaft assembly comprising a cannulated outer shaft having an outer shaft flexible section, and a cannulated inner shaft having an inner shaft flexible section. The outer shaft flexible section and the inner shaft flexible section each comprises a respective plurality of segmented floating links. The inner shaft is configured and dimensioned to be inserted into the outer shaft in an operating position in which at least a portion of the inner shaft flexible section is surrounded by at least a portion of the outer shaft flexible section, such that the inner shaft flexible section and the outer shaft flexible section can flex in unison, and the inner shaft is rotatable within the outer shaft. The instrument shaft may be used with a surgical tissue shaver. Methods of use are also provided.

## Description

### Background

Various surgical instruments use a shaft formed by concentric tubes. In some cases, one or both of the concentric tubes is a flexible tube. For example, surgical shavers sometimes have a shaft assembly formed by concentric wound wires or helical ribbons of material. Such wound wires or ribbons extend continuously to form a flexible section of the shaft, and act as a spring to restore the shaft to a predetermined orientation (typically straight) in the absence of loads on the shaft.

Segmented flexible tubes (i.e., those with fully separate "floating" links rather than a continuous wire or ribbon) are also known as an alternative for providing a flexible shaft section. Segmented flexible tubes are formed by interconnected flexible links that allow the flexible section to bend freely, without any inherent restoring force to hold them in any particular position.

Under conventional thinking, the inability of a segmented flexible tube to naturally assume a particular orientation (i.e., the lack of inherent resilience) has been considered a problem to be overcome, typically by coupling the segmented flexible tube with a resilient polymeric liner or sheath, or using the segmented flexible tube in a concentric relation with a conventional flexible tube having a spring-like flexible section.

With this conventional understanding in place, the concept of using multiple concentric segmented flexible tubes has not been developed, and its benefits have remained undiscovered.

### Summary

In a first exemplary embodiment, there is provided a surgical instrument shaft assembly comprising: an outer shaft extending from a proximal outer shaft end to a distal outer shaft end and having an outer shaft cannula extending from the proximal outer shaft end to the distal outer shaft end, wherein at least a portion of the outer shaft comprises an outer shaft flexible section located between the proximal outer shaft end and the distal outer shaft end, and the outer shaft flexible section comprises a plurality of first segmented floating links; and an inner shaft extending from a proximal inner shaft end to a distal inner shaft end and having an inner shaft cannula extending from the proximal inner shaft end to the distal inner shaft end, wherein at least a portion of the inner shaft comprises an inner shaft flexible section located between the proximal inner shaft end and the distal inner shaft end, and the inner shaft flexible section comprises a plurality of second segmented floating links. The inner shaft is configured and dimensioned to be inserted into the outer shaft in an operating position in which: at least a portion of the inner shaft flexible section is surrounded by at least a portion of the outer shaft flexible section, such that the inner shaft flexible section and the outer shaft flexible section can flex in unison, and the inner shaft is rotatable within the outer shaft.

In some embodiments, the distal outer shaft end comprises an outer cutter head extending along a first longitudinal axis and having an outer cutter opening in fluid communication with the outer shaft cannula and facing perpendicular to the first longitudinal axis, and the distal inner shaft end comprises an inner cutter head extending along a second longitudinal axis and having an inner cutter opening in fluid communication with the inner shaft cannula and facing perpendicular to the second axis direction. In such embodiments, in the operating position, at least a portion of the inner cutter head is surrounded by at least a portion of the outer cutter head with the first longitudinal axis collinear with the second longitudinal axis, and the inner cutter opening is rotatable about the second longitudinal axis, upon rotating the inner shaft relative to the outer shaft, to selectively move between a first state of alignment between the inner cutter opening and the outer cutter opening, and a second state of alignment between the inner cutter opening and the outer cutter opening, the second state of alignment being different from the first state of alignment.

In another exemplary embodiment, there is provided a method for operating a surgical instrument comprising a guide tube, an outer shaft having an outer shaft cannula and a first plurality of first segmented floating links defining an outer shaft flexible section, and an inner shaft having an inner shaft cannula and a plurality of second segmented floating links defining an inner shaft flexible section. The method comprises: inserting the inner shaft and the outer shaft into the guide tube; positioning the inner shaft flexible section concentrically within the outer shaft flexible section; rotating the outer shaft relative to the guide tube; and rotating the inner shaft relative to the outer shaft.

### Brief Description of the Drawings

Figure 1 is an assembled view of an exemplary embodiment of a surgical instrument shaft assembly.
Figure 2 is an exploded view of the embodiment of Figure 1.
Figure 3 is a detail view of portions of an exemplary embodiment of an outer shaft including a tissue shaving instrument.
Figure 4 is a detail view of portions of an exemplary embodiment of an inner shaft including a tissue shaving instrument.
Figure 5 is an isometric view of an exemplary shaft having a flexible section comprising a plurality of segmented floating links.
Figure 6 is an exploded view of an example of a prior art surgical instrument shaft assembly.
Figure 7 illustrates an exemplary embodiment of a surgical instrument shaft assembly having a tissue shaving instrument, shown with the guide tube in cross-section.
Figure 8 illustrates another exemplary embodiment of a guide tube.
Figure 9 illustrates another exemplary embodiment of a guide tube.
Figures 10a-10c illustrate a portion of the embodiment of Figure 7, showing the inner cutting head in three different positions relative to the outer cutting head.
Figures 11a and 11b illustrate a portion of the embodiment of Figure 7, showing the outer cutting head in two different positions relative to the guide tube.
Figure 12 shows a first exemplary alternative mechanism for holding an outer shaft of a surgical instrument shaft assembly.
Figure 13 shows a second exemplary alternative mechanism for holding an outer shaft of a surgical instrument shaft assembly.
Figure 14 illustrates the embodiment of Figure 7 in an exemplary use.

### Description of Exemplary Embodiments

The following is a description of exemplary embodiments of a surgical instrument shaft assembly that may be used with surgical instruments configured to perform various surgical procedures. In the examples below, the surgical instrument is a shaver, such as a tissue shaver that might be used during arthroscopic surgical procedures to collect body tissue (bone, ligament, muscle, lesions, etc.). Other instrument may include reamers, drills, screwdrivers, and so on.

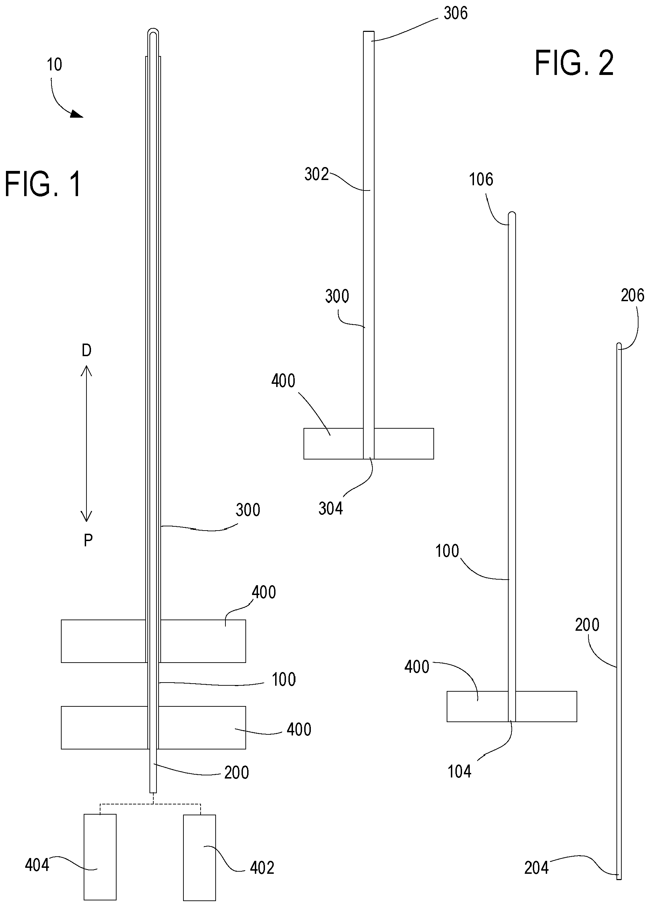

Referring to Figures 1 and 2, the surgical instrument shaft assembly 10 includes an outer shaft 100, an inner shaft 200, and an optional guide tube 300. One or more of the outer shaft 100, inner shaft 200 and guide tube 300 may include a handle 400, clamp mount or other mechanism to facilitate movement and control. In the shown example, the outer shaft 100 and guide tube 300 have respective handles, which may include features to lock them together for common movement, as known in the art. In this example, the inner shaft 200 may be selectively secured to a drive accessory 402 such as a drive motor for providing unidirectional or oscillating rotation, or a suction accessory 404 such as a syringe or vacuum pump.

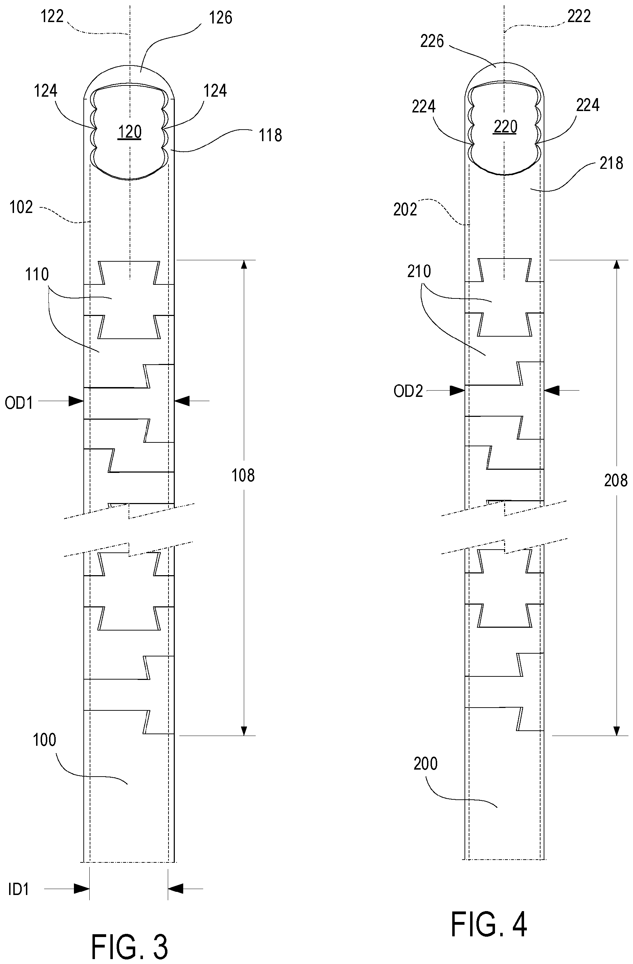

The outer shaft 100 comprises a generally tubular structure made of surgical-grade material, such as stainless steel or the like. The outer shaft 100 has an outer shaft cannula 102 (Fig. 3) extending continuously from a proximal outer shaft end 104 to a distal outer shaft end 106. As used herein, the terms "proximal" and "distal" refer to the near and far directions, respectively, of the assembled system during use, in which case the distal direction is facing and extending towards the surgical patient, while the proximal direction is facing and extending towards the surgeon handling the device. The proximal outer shaft end 104 is open to receive the inner shaft 200, as explained below. The distal outer shaft end 106 may be open at its end and/or sides to permit functionality of the inner shaft 200. At least a portion of the outer shaft 100 comprises an outer shaft flexible section 108 located between the proximal outer shaft end 104 and the distal outer shaft end 106. The outer shaft flexible section 108 comprises a plurality of first segmented floating links 110, as discussed in more detail below.

The inner shaft 200 extends from a proximal inner shaft end 204 to a distal inner shaft end 206. The inner shaft 200 may have an inner shaft cannula 202 (Fig. 4) extending from the proximal inner shaft end 204 to the distal outer shaft end 206, but the cannula 202 may be omitted in some such as those in which a tissue transport function is not required (e.g., a screwdriver or the like). At least a portion of the inner shaft 200 comprises an inner shaft flexible section 208 located between the proximal inner shaft end 204 and the distal inner shaft end 206. The inner shaft flexible section 208 comprises a plurality of second segmented floating links 210, as discussed in more detail below.

The inner shaft 200 is dimensioned to fit concentrically within the outer shaft 100 to place the inner shaft 200 and outer shaft 100 into an operating position. In the operating position, the inner shaft flexible section 208 at least partially overlaps with the outer shaft flexible section 108, such that the overlapping portions of the inner shaft flexible section 208 and the outer shaft flexible section 108 can flex in unison relative to remaining portions of the outer shaft 200 and inner shaft 100.

Also, when in the operating position, the inner shaft 200 is rotatable within the outer shaft 100. As used herein, the term "rotatable," as it relates to relative movement between the outer shaft 100, inner shaft 200 and guide tube 300, refers to rotation about the respective central cylindrical axes of the parts. Rotation can be complete (i.e. relative rotation about a range of 360°) or limited to a specific range (e.g., relative rotation about a range of less than 360°, as may be determined by adding rotational travel stops to the parts).

Relative rotation between the outer shaft 200 and the inner shaft 200 is provided by forming the inner shaft 200 with an outer diameter OD2 that is less than an inner diameter ID1 of the outer shaft cannula 102 at all locations along the overlapping lengths of the inner shaft 200 and outer shaft 100. It will be appreciated that one or both of the inner shaft outer diameter OD2 and outer cannula inner diameter ID1 may vary as a function of position between their respective proximal and distal ends, such as by having diameter changes occurring at discrete steps or gradual changes. In a preferred embodiment, however, the outer diameter OD2 of the inner shaft 200 is generally constant, and so too is the inner diameter ID1 of the outer shaft cannula 102.

When the surgical instrument shaft assembly 10 is provided with a guide tube 300, the guide tube comprises a rigid tubular structure formed of stainless steel, plastic, or any other material having sufficient rigidity and sterility for the desired application. The guide tube 300 extends from a proximal guide tube end 304 to a distal guide tube end 306, and a guide tube cannula 302 extends the full distance from the proximal guide tube end 304 to a distal guide tube end 306. The guide tube cannula 302 is dimensioned to concentrically receive the outer shaft 100, which can be accomplished by making the guide tube cannula 302 with an inner diameter that is larger than the corresponding outer diameter OD1 of the outer shaft 100. The guide tube cannula 302 may have a constant inner diameter, and the outer shaft 100 may have a constant outer diameter OD1, but neither feature is required in all embodiments. In addition, the guide tube 300 and outer shaft 100 may be configured to allow relative rotation of the outer shaft 100 and the guide tube 300, such as by providing sufficient open space between their respective facing surfaces, but this is not strictly required in all embodiments.

In the operating position, the proximal inner shaft end 204 may extend in the proximal direction P from the proximal outer shaft end 104, such as shown in Figure 1. This facilitates both rotation of the inner shaft 200 relative to the outer shaft 100, and removal of the inner shaft 200 from the outer shaft 100 if necessary. Similarly, and also shown in Figure 1, when a guide tube 300 is used, one or both of the proximal outer shaft end 104 and proximal inner shaft end 204 may extend in the proximal direction P from the proximal guide tube end 304. Likewise, the distal inner shaft end 206 may extend in the distal direction D from one or both of the distal outer shaft end 106 and the distal guide tube end 306, and the distal outer shaft end 106 may extend in the distal direction D from the distal guide tube end 306.

As noted above, the outer shaft flexible section 108 comprises a plurality of first segmented floating links 110, and the inner shaft flexible section 208 comprises a plurality of second segmented floating links 210. Each segmented floating link 110, 210 is a discrete structure that is not joined by a continuous material connection to each adjacent segmented floating link 110, 210. Furthermore, each segmented floating link 110, 210 has interlocking structures that loosely engage each adjacent segmented floating link 110, 210 in a manner that allows the adjacent segmented floating links 110, 210 to freely move relative to each other through a limited range of travel, but does not allow the adjacent segmented floating links 110, 210 to completely separate from one another.

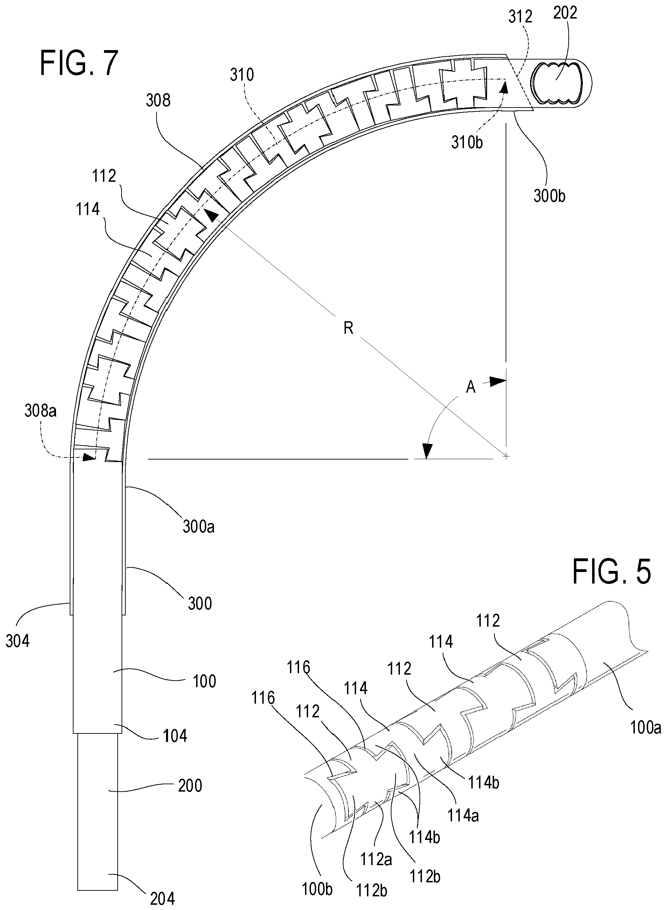

Referring to Figure 5, the exemplary segmented floating links 110 of the outer shaft 100 comprise alternating dovetail links 112 and pin links 114. Each dovetail link 112 has a central body 112a that extends circumferentially to define a closed loop that forms part of the outer surface of the outer shaft 100 and outer shaft cannula 102, and at least two dovetails 112b extending in each of the distal direction D and proximal direction P from the central body 112a. Each dovetail 112b is defined by inner and outer circumferential surfaces having a trapezoidal shape as viewed perpendicular to the outer shaft 100, with the narrow side of the trapezoid joined to the central body 112a. The inner and outer trapezoidal surfaces form portions of the outer shaft cannula 102 and outer surface of the outer shaft 100, respectively.

Each pin link 114 has a central body 114a that extends circumferentially to define a closed loop that forms part of the outer surface of the outer shaft 100 and outer shaft cannula 102, and at least two pins 114b extending in each of the distal direction D and proximal direction P from the central body 114a. Each pin 114b is defined by inner and outer circumferential surfaces having a trapezoidal shape as viewed perpendicular to the outer shaft 100, with the narrow side of the trapezoid joined to the central body 114a. The inner and outer trapezoidal surfaces form portions of the outer shaft cannula 102 and outer surface of the outer shaft 100, respectively.

The dovetails 112b and pins 114b are shaped and dimensioned to engage one another to prevent the dovetail links 112 from separating from the pin links 114. However, as best shown in Figure 5, a continuous clearance gap 116 is provided between each adjacent dovetail link 112 and pin link 114. The clearance gap 116 extends continuously around the circumference of the outer shaft 100, and thus there is no material connection between each adjacent dovetail link 112 and pin link 114. The interlocking shapes of the dovetails 112b and pins 114b, in combination with the clearance gap 116, provides a "floating connection" that allows each adjacent dovetail link 112 and pin link 114 to move relative to each other through a limited range of motion, both in the axial direction (i.e., along the proximal-to-distal direction) and in the circumferential direction (i.e. the direction of rotation about the central axis of the outer shaft 100, which is defined, in the outer shaft flexible section 108, by the respective central axis of each link 112, 114).

The ends of the outer shaft flexible section 108 are defined by dovetails 112b and/or pins 114b formed on or attached to adjacent rigid portions 110a, 110b of the outer shaft 110.

It will be appreciated that the pins 114b and dovetails 112b can have essentially the same shape (i.e., trapezoidal in this case), but this is not strictly required. For example, the pins 114 may have a trapezoidal shape with rounded corners, or an ovate or circular shape. Also, both the dovetails 112b and the pins 114b may have interlocking shapes other than trapezoidal shapes.

Segmented floating links of various types are known in the art, and other constructions may be used in other embodiments. Further examples of segmented floating links are described, for example, in United States Patent No. 8,366,559, which is incorporated herein by reference in its entirety.

The dovetail links 112 and pin links 114 may be constructed using any suitable manufacturing technique. In a preferred embodiment the dovetail links 112 and pin links 114 are formed from a single continuous tube by cutting the clearance gaps 116 using a laser.

Regarding the inner shaft 200, the second segmented floating links 210 defining the inner shaft flexible section 208 are constructed in a manner similar to the first segmented floating links 110 of the outer shaft flexible section 108, and the foregoing description of the first segmented floating links 110 is sufficient to describe exemplary embodiments and alternatives for the second segmented floating links 210. It will also be understood that, in any given embodiment, the first segmented floating links 110 and second segmented floating links 210 can have similar constructions (e.g., all trapezoidal dovetails and pins) or they may have dissimilar constructions (e.g., trapezoidal dovetails and pins for the first segmented floating links 110 and interlocking circular dovetails and pins for the second segmented floating links 210). Other alternatives and embodiments will be apparent to persons of ordinary skill in the art in view of the present disclosure.

In the shown exemplary embodiment, the overlapping portions of the outer shaft flexible section 108 and the inner shaft flexible section 208 also do not include any resilient supporting structures. For example, neither the outer shaft flexible section 108 nor the inner shaft flexible section 208 includes an internal or external polymeric or elastic tube, coil spring, wire spring, or other structure to bias the assembled flexible sections 108, 208 into a particular orientation (e.g., straight). Thus, in this embodiment, the combined inner shaft 200 and outer shaft 100 can flex freely and essentially without resistance to any particular orientation.

This arrangement is contrary to conventional concentric surgical instrument shaft constructions in which an inner shaft is rotatably positioned within an outer shaft (e.g., as might be used for a tissue shaver). In conventional devices, one or both of the inner and outer shafts is either formed by a rigid structure, or with a resilient spring or the like to bias the flexible sections of the shafts to a particular position. Such rigidity or biasing force was considered favorable to allow a surgeon to guide the working end of the shaft assembly to a desired location, and hold the working end near or against tissue to perform a procedure. Additionally, by forming one or both of the inner and outer shafts as a rigid member or with a resilient bias, it was not necessary to provide other mechanisms to help guide and hold the working end of the shaft assembly, which avoided adding additional parts that would increase the diameter of the assembly. Thus, the assembly could be used in narrow or confined spaces, and would require less tissue trauma to the patient.

The inventor has discovered, however, that conventional concentric surgical instrument shafts, particularly those with flexible sections, have a significant shortcoming. Specifically, the resilient force generated by the flexible shaft (whether it is the inner shaft, the outer shaft, or both) creates friction between the two shafts, which increases the drive force necessary to rotate the inner shaft relative to the outer shaft, increases heat generation and wear, and potentially leads to erosion of fragments of the shafts that can end up in the removed tissue. Furthermore, the resilience of the flexible shaft(s) can generate a "bouncing" effect as the inner shaft rotates, leading to reduced positional control and less consistent application of the working end to the target tissue.

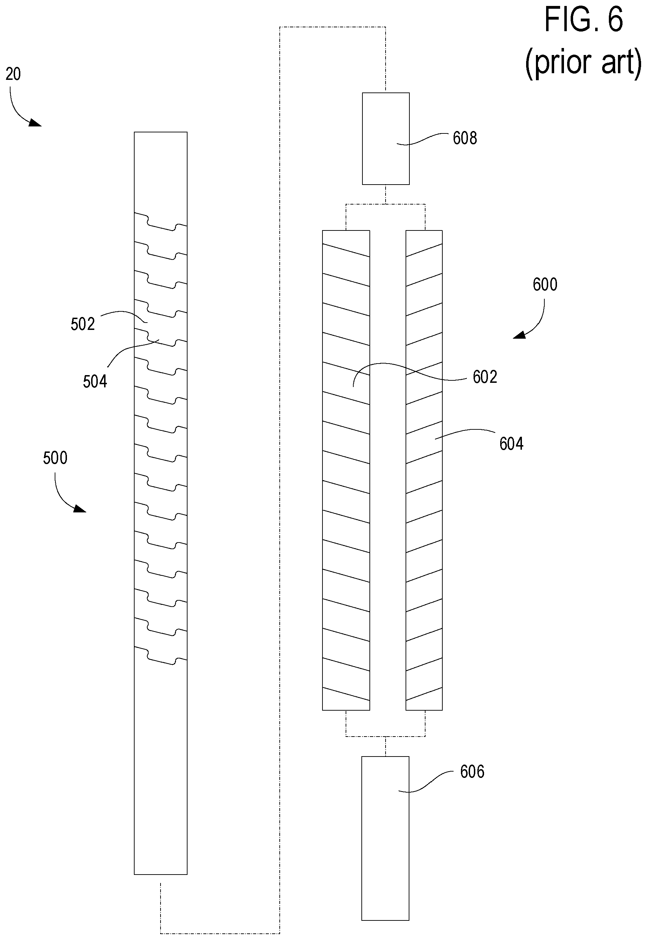

Still further, it has been found that conventional resilient shafts that are made of one or more layers of continuous coiled spring (e.g., as in a typical shaver shaft assembly) have very particular and distinct problems. An example of a prior art shaft of this sort is shown in Figures 6 and 7. Here, the shaft assembly 20 comprises an outer shaft 500, and an inner shaft 600. The outer shaft 500 is a tubular structure having a flexible section formed by single continuous helical ribbon 502 of material, which may have interlocking shapes 504 joining each adjacent wrap of the ribbon 502. This structure is typically formed by using a laser to cut a single continuous helical slot along the length of an otherwise continuous tube of material. The continuous ribbon 502 allows resilient flexing of the outer shaft 500, but rotation of the outer shaft 500 causes the helical ribbon 502 to expand and contract, which results in slight increases and decreases in the length and diameter of the outer shaft 500, depending on the direction of rotation (the interlocking shapes 504 limit, but do not prevent such shape changes). The inner shaft 600 is formed by concentric and oppositely twisting flexible springs 602, 604, each of which is formed from a single ribbon of material that is typically formed by laser cutting a single helical slot along the length of an otherwise continuous tube of material (in some cases, more concentric flexible springs may be added, and one or more of the springs may be formed by a bent wire). The concentric springs 602, 604 must be formed separately, and joined, such as by welding, to a rigid drive tube ends 606 and a rigid working tube end 608. The oppositely-twisted springs 602, 604 are necessary to avoid expansion and contraction - particularly increases in the diameter - of the flexible section of the inner shaft 600. This is important to prevent the inner shaft 600 from expanding and binding with the outer shaft during rotation in one direction or the other.

The conventional device shown in Figure 6 suffers from two problems. First, it is expensive and difficult to manufacture. Second, relative rotation of the parts generates friction and torque loads on the inner and outer shafts 600, 500, which leads to a spring-like and inefficient rotational behavior at the working tube end 608. Specifically, it has been discovered that applying a drive force to the drive tube end 606 while the working tube end 608 faces rotational resistance (e.g., while shaving tissue or driving a screw) causes the working tube end 608 to hesitate before moving in unison with the drive tube end 606. During unidirectional rotation, this results in angular velocity changes at the working tube end 608 as resistance against the tissue changes. During bi-directional oscillating rotation, and especially the high oscillation rates used in tissue shavers (e.g., 5,000 to 12,000 cycles per minute), this results in lagging and irregular application of the shaver to the tissue. Without being bound to any theory of operation, it is believed this happens because torque loads bias the flexible section of the inner tube 600 to assume a helical shape, which causes the working tube end 608 to move out of unison with the drive tube end 606.

The detrimental effect of the lagging spring-like behavior at the working tube end 608 becomes apparent when comparing the operation of a conventional tissue shaver with the operation of a tissue shaver having a shaft assembly according to the foregoing embodiments. In particular, it has been found that embodiments of the invention provide faster and more effective tissue removal.

Furthermore, the utility and benefits of instrument shafts according to embodiments of the present invention were unexpected. First, without including any kind of smooth layer of material (e.g., a flexible sheath) between the outer shaft 100 and the inner shaft 200, it would be expected that the segmented floating links 110, 210 of each shaft could contact each other at their edges at the gaps formed by the clearance gaps 116. This would lead to hard strikes that generate spikes in the torsional resistance, excess friction, possible damage to the segmented floating links 110, 210, or even complete prevention of relative rotation between the outer shaft 100 and the inner shaft 200 (i.e. jamming). Such potential problems would be even more expected in instruments having high oscillation frequencies, such as tissue shavers. Furthermore, the improved performance provided by two unbiased flexible shafts, which is believed to be obtained by omitting or at least minimizing any spring-like resilient forces in the flexible shafts (particularly in tissue shavers, but also potentially in other applications) was heretofore undiscovered and unexpected. Thus, while not strictly required in all cases, it is preferred for the inner shaft flexible section 208, or more preferably both the outer shaft flexible section 108 and the inner shaft flexible section 208, to be devoid of any kind of flexible sheath (i.e., no internal or external liner that provides a resilient restoring force).

It has also been surprisingly discovered that omitting the flexible sheath from the inner, or inner and outer, flexible sections 108, 208 also provides a benefit of reducing removed tissue into smaller pieces, which helps facilitate removal. Without being bound to any theory of operation, it is believed that such size reduction is caused by particles being ground between adjacent floating links 110, 210 of each flexible section, and possibly also by grinding between the outer surfaces of the inner floating links 210 and the inner surfaces of the outer floating links 110.

Referring to Figure 7, an example of a guide tube 300 is shown in greater detail. In this case, the guide tube 300 has a bent section 308 having a bend angle A. The bend angle A is measured as the angle between the central cylindrical axis 310 at each end 310a, 310b of the bent section 308. In the shown example, bent section 308 extends between a first cylindrical region 300a of the guide tube 300 and second cylindrical region 300b of the guide tube 300. The second cylindrical region 300b terminates at an opening 312 that may be shaped to help support the first shaft 100 and second shaft 200 during operation. For example, the shown guide tube opening 312 is beveled to provide additional support against forces applied against the longer side of the bevel. Other alternatives and embodiments will be apparent to persons of ordinary skill in the art in view of the present disclosure.

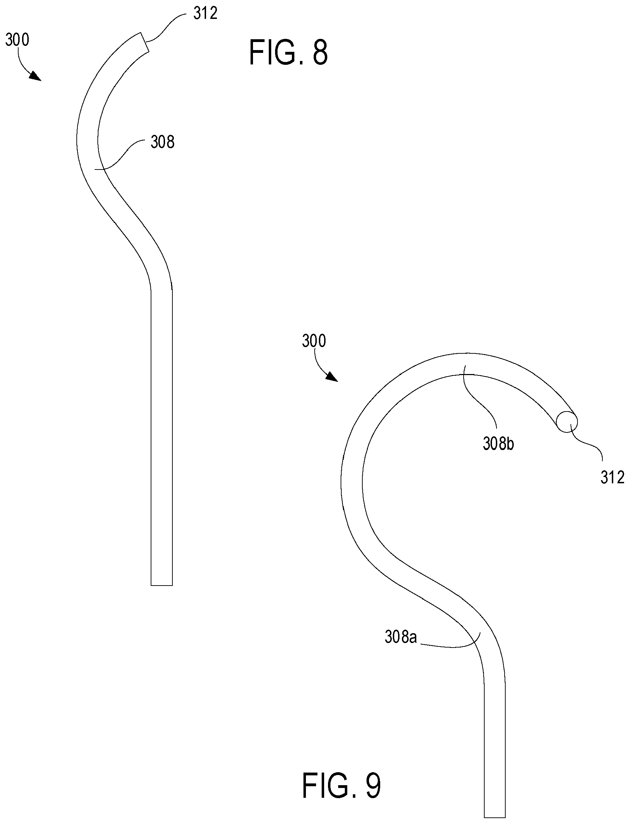

The illustrated bent section 308 is shaped as a circular segment that lies in a single plane and is defined by a radius R. In other cases, the bent section 308 may have an elliptical shape, a hyperbolic shape, a shape that bends in three dimensions, or any other shape that may be desired while still allowing proper operation of the outer shaft 100 and inner shaft 200. Other examples are shown in Figure 8 and 9. In Figure 8, the bent section 308 has a simple compound curve lying in a single plane (e.g., a flat S-shape). In Figure 9, the bent section 308 comprise a compound curve having a first portion 308a lying in one plane, and a second portion that curves along a helical path to terminate at a cylindrical outlet opening 312 that faces perpendicular to the plane of the first portion 308a.

As noted above, the bent section 308 may have any shape that allows suitable rotation of the inner shaft 200 relative to the outer shaft 100, and (if required) the outer shaft 100 relative to the guide tube 300. It has been found that embodiments may have relatively large bend angles A and relatively small bend radii R as compared to conventional devices, such as conventional tissue shavers. For example, in one embodiment the bend angle A may be 90° or greater (e.g., 180° or even greater), as compared to a typical conventional bend angle of 30° or less. As another example, the bend radius R may be on inch or less, as compared to a larger conventional bend radii. Still other embodiments may have both a bend angle of 90° or more, and a bend radius R of one inch or less.

It has also been found that embodiments also may have smaller diameters than conventional concentric flexible shaft assemblies. For example, the first segmented floating links 110 of the outer shaft 100 may have a diameter OD1 of 0.150 inches or less (e.g., 0.138 inches), and the second segmented floating links 210 of the second shaft 20 may have an outer diameter OD2 of 0.120 inches or less (e.g., 0.105 inches).

These improvements to bend angle A, bend radius R and outer diameter OD1, OD2 can be used to provide surgical instrument shaft assemblies 10 that are significantly more useful than conventional devices. For example, embodiments may allow greater access to certain regions of the body, and provide less extensive trauma to the patient.

Referring back to Figure 7, it will be appreciated that, when the parts are in the operating position, the outer shaft flexible section 108 and inner shaft flexible section 208 are located in the guide tube cannula 302 within the bent section 308. Specifically, at least a portion of the outer shaft flexible section 108 and at least a portion of the inner shaft flexible section 208 preferably extend throughout the entire bent section 308 to allow free rotation of the outer shaft 100 relative to the guide tube 300, and the inner shaft 200 relative to the outer shaft 100. One or both of the outer shaft flexible section 108 and inner shaft flexible section 208 also may extend beyond the bent section 308 of the guide tube 300.

As also shown in Figure 7, the proximal outer shaft end 104 may extend in the proximal direction P from the proximal guide tube end 304, and the proximal inner shaft end 204 may extend in the proximal direction P from the proximal outer shaft end 104. This facilitates independent operation and movement of the guide tube 300, outer shaft 100, and inner shaft 200, but is not strictly required in all cases.

Embodiments such as those discussed above may be used with various surgical instruments. The examples herein are shown, in a non-limiting manner, in the context of a tissue shaver. In this configuration, the distal outer shaft end 106 comprises an outer cutter head 118 having an outer cutter opening 120. The outer cutter head 118 comprises a generally cylindrical body that extends along a first longitudinal axis 122, and the outer cutter opening 120 faces perpendicular to the first longitudinal axis 122. The outer cutter opening 120 is in fluid communication with the outer shaft cannula 102.

Similarly, the distal inner shaft end 206 comprises an inner cutter head 218 having an inner cutter opening 220. The inner cutter head 218 comprises a generally cylindrical body that extends along a second longitudinal axis 222, and the inner cutter opening 220 faces perpendicular to the second longitudinal axis 222. The inner cutter opening 220 is in fluid communication with the inner shaft cannula 202.

In the operating position, the distal outer shaft end 106 at least partially surrounds the distal inner shaft end 206, with the first longitudinal axis 122 being collinear with the second longitudinal axis 222. In this position, the inner cutter head 218 is at least partially surrounded by the outer cutter head 118, and rotating the inner shaft 200 relative to the outer shaft 100 causes the inner cutter head 218 to rotate, relative to the outer cutter head 118, about the collinear first and second longitudinal axes 122, 222.

The outer cutter opening 120 and inner cutter opening 220 are positioned on their respective cutter heads such that rotation of the inner cutter head 218 relative to the outer cutter head 118 causes the inner cutter opening 220 to move between different states of alignment relative to the outer cutter opening 120. Such movement causes the edges of the cutter openings 120, 220 to close together to sever adjacent tissue, and then separate to form a fluid communication path into the inner shaft cannula 202 to allow tissue to be removed by suction or the like.

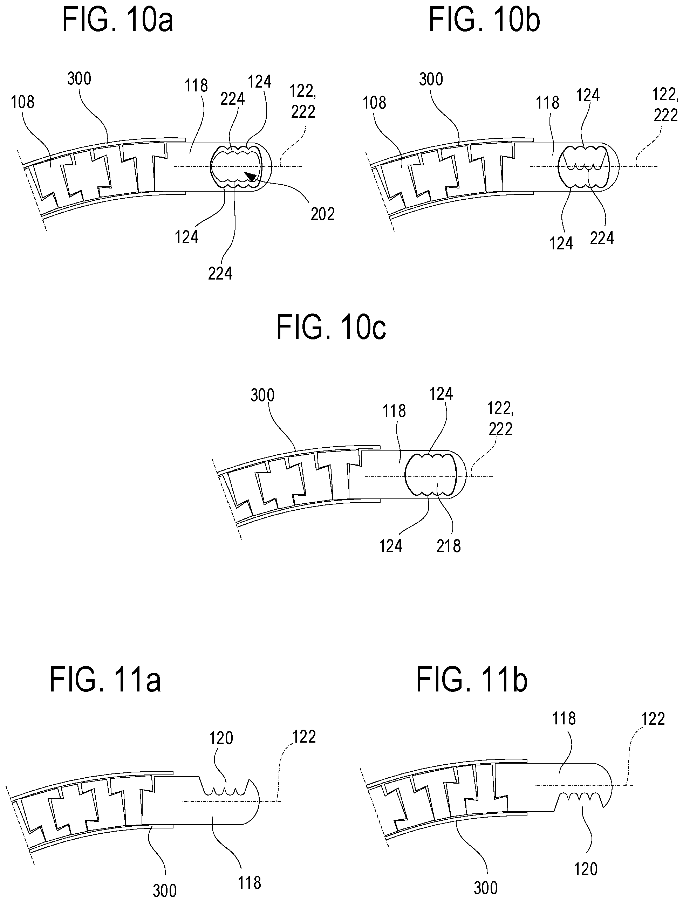

For example, as shown in Figures 10a and 10b, the inner cutter opening 220 may be movable relative to the outer cutter opening 120 between a first state of alignment (Figure 10a) in which the outer cutter opening 120 and inner cutter opening 220 form a fluid communication path of a first cross-sectional size into the inner shaft cannula 202, and a second state of alignment (Figure 10b), in which the outer cutter opening 120 and inner cutter opening 220 form a fluid communication path of a second cross-sectional size into the inner shaft cannula 202, with the second cross-sectional size being less than the first cross-sectional size. In addition, further rotation of the inner cutter head 218 causes the outer cutter opening 120 and inner cutter opening 220 to attain a third state of alignment (Figure 10c), in which the inner cutter opening 220 is does not overlap with the outer cutter opening 120, and thus the outer cutter opening 120 and inner cutter opening 220 do not form a fluid communication path to the inner shaft cannula 202. As the cutter openings 120, 220 move to the third state of alignment, side edges 124, 224 of the cutter openings 120, 220 approach each other to pinch and sever adjacent tissue. To this end, the edges 124, 224 may be sharpened, and may include one or more teeth or serrations to help assist with pinching and severing the tissue.

In use, the inner shaft 200 may be selectively rotated in one direction or the other, or cyclically rotated in both directions in an oscillating manner. In typical use, the inner cutter head 218 is oscillated back and forth between the fully-open position as shown in Figure 10a and the fully-closed position as shown in Figure 10c. During each complete oscillation cycle, the inner cutter opening 220 moves back and forth completely across the outer cutter opening 120 to cut tissue on both sides of the outer cutter opening 120. However, this is not strictly required. Also, it is not strictly necessary for the outer cutter head 118 and inner cutter head 220 to achieve the third state as shown in Figure 10c in all instances or during each operation cycle.

The outer shaft 100 and inner shaft 200 also may include features to ensure that they remain in the operating position to properly align the outer cutter opening 120 and inner cutter opening 220. For example, the outer cutter head 118 may include a first end stop 126 that abuts a second end stop 226 on the inner cutter head 218, to prevent motion of the inner cutter head 218 beyond the desired position. In this case, the end stops 126, 226 comprise hemispherical end walls of the respective shaft, but other configurations are possible.

Other embodiments also may use different types of tissue shaver devices. For example, the inner cutter head 218 may be replaced by a helical auger or a round cutting burr, and the outer cutter head 118 may comprise a simple circular opening against which the auger or burr acts to remove tissue. Other alternatives and embodiments will be apparent to persons of ordinary skill in the art in view of the present disclosure.

Referring now to Figures 11a and 11b, embodiments may be configured such that the outer shaft 100 can be rotated relative to the guide tube 300. For example, in Figure 11a, the outer shaft 100 is rotated relative to the guide tube 300 to orient the outer cutter head 118 with the outer cutter opening 120 facing in the distal direction D, whereas in Figure 11b, the outer shaft 100 is rotated relative to the guide tube 300 to orient the outer cutter head 118 with the outer cutter opening 120 facing in the proximal direction P. Any other orientation about the first longitudinal axis 122 is also possible, or travel stops may be provided to prevent rotation or to limit the range of rotation. Such repositioning is useful to increase surgeon's ability to use the instrument to shave tissue in different directions. However, this feature is not strictly required in all embodiments.

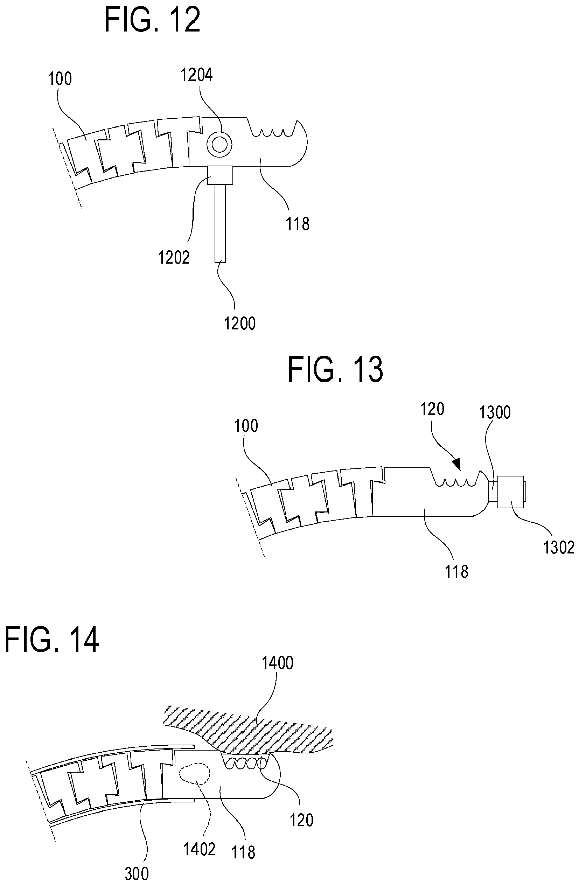

As noted above, a guide tube 300 may be used to hold the working ends of the inner shaft 100 and outer shaft 200 at a desired location. However, other embodiments may use other mechanisms to perform this function. For example, Figure 12 shows an alternative embodiment in which the outer cutter head 118 (and thus the inner cutter head 218) is held in a desired position by a rod 1200 that is secured to the outer cutter head 118. In this case, the rod 1200 is threaded into a receptacle 1202 located on the outer cutter head 118, and the outer cutter head 118 also may have one or more additional receptacles 1204 to allow the surgeon to select a particular holding location. The rod 1200 also may be secured by a permanent attachment (e.g., welding), by a clamp that allows the rod 1200 orientation to be changed, and so on.

The embodiment of figure 13 shows another mechanism for holding the outer cutter head 118. In this case, the outer cutter head 118 has an axial projection 1300 that fits into a bushing 1302. The bushing 1302 may be secured to the patient's body adjacent to the tissue being removed (e.g., by wires or screws), or held in place with a rod such as discussed above. The projection 1300 and bushing 1302 may be configured to allow relative rotation between the axial projection 1300 and bushing 1302, to provide the ability to selectively rotate the outer shaft 100 and reorient the outer cutter opening 120, without having to adjust the bushing 1302.

In still other embodiments, the outer cutter head 118 may be held in place by engaging an existing surgical implant (e.g., secured to an existing hole in a trauma plate or implant to perform further procedures in the area), held by forceps or the like, and so on. Other alternatives and embodiments will be apparent to persons of ordinary skill in the art in view of the present disclosure.

Embodiments of surgical instrument shaft assemblies such as described herein may be used in various ways, with the particular details possibly varying depending on the type of surgical instrument, location of the surgical site, type of tissue being address, and the purpose of the procedure.

Figure 14 shows a surgical instrument shaft assembly in use with a tissue shaver. In this case, a surgical procedure is performed by inserting the inner shaft 200 and outer shaft 100 into the guide tube 300, positioning the inner shaft flexible section 208 concentrically within the outer shaft flexible section 108, rotating the outer shaft 200 relative to the guide tube 300 to place the outer cutter opening 120 at the desired orientation, and rotating the inner shaft 200 relative to the outer shaft 100 in a cyclical or unidirectional manner to shave tissue 1400. The shaved tissue 1402 is then pulled by suction down the inner shaft cannula 202 for collection and/or disposal. During use, the surgeon can periodically rotate the outer shaft 100 to reorient the outer cutter opening 120 as desired to address other regions of tissue 1400. Other alternatives and embodiments will be apparent to persons of ordinary skill in the art in view of the present disclosure.

The present disclosure provides a number of exemplary embodiments of the invention defined by the appended claims. The description of such embodiments is not intended to limit the scope of the claims beyond what is defined in the claims. It will also be understood that, while embodiments may provide particular advantages in certain cases, the scope of the claims is not limited to embodiments providing any particular advantage or functionality. It will further be appreciated that other embodiments encompassed by the claims may diverge from those described herein in both appearance and functionality, and the various features of particular described herein may be used with other embodiments without departing from the scope of the claims.

## Claims

1. A surgical instrument shaft assembly comprising:
an outer shaft (100) extending from a proximal outer shaft end (104) to a distal outer shaft end (106) and having an outer shaft cannula (102) extending from the proximal outer shaft end (104) to the distal outer shaft end (106), wherein at least a portion of the outer shaft (100) comprises an outer shaft flexible section (108) located between the proximal outer shaft end (104) and the distal outer shaft end (106), and the outer shaft flexible section (108) comprises a plurality of first segmented floating links (110); and
an inner shaft (200) extending from a proximal inner shaft end (204) to a distal inner shaft end (206) and having an inner shaft cannula (202) extending from the proximal inner shaft end (204) to the distal inner shaft end (206), wherein at least a portion of the inner shaft (200) comprises an inner shaft flexible section (208) located between the proximal inner shaft end (204) and the distal inner shaft end (206), and the inner shaft flexible section (208) comprises a plurality of second segmented floating links (210);
wherein the inner shaft (200) is configured and dimensioned to be inserted into the outer shaft (100) in an operating position in which:
at least a portion of the inner shaft flexible section (208) is surrounded by at least a portion of the outer shaft flexible section (108), such that the inner shaft flexible section (208) and the outer shaft flexible section (108) can flex in unison, and
the inner shaft (200) is rotatable within the outer shaft (100).

2. The surgical instrument shaft assembly of claim 1, wherein:
the plurality of first segmented floating links (110) comprises alternating first dovetail links (112) and first pin links (114), wherein each adjacent first dovetail link and first pin link are connected by a respective floating connection; and
the plurality of second segmented floating links (210) comprises alternating second dovetail links and second pin links, wherein each adjacent second dovetail link and second pin link are connected by a respective floating connection.

3. The surgical instrument shaft assembly of any of the preceding claims, wherein, in the operating position, the proximal inner shaft end (204) extends in a proximal direction (P) from the proximal outer shaft end (104).

4. The surgical instrument shaft assembly of any of the preceding claims, further comprising a guide tube (300) extending from a proximal guide tube end (304) to a distal guide tube end (306) and having a guide tube cannula (302) extending from the proximal guide tube end (304) to the distal guide tube end (306), and wherein, in the operating position, at least a portion of the outer shaft (100) and at least a portion of the inner shaft (200) are positioned within the guide tube cannula (302).

5. The surgical instrument shaft assembly of claim 4, wherein at least a portion of the guide tube (300) comprises a bent section (308), and, in the operating position:
at least a portion of the outer shaft flexible section (108) extends throughout the entire bent section (308);
at least a portion of the inner shaft flexible section (208) extends throughout the entire bent section (308); and
the outer shaft (100) is rotatable within the guide tube (300).

6. The surgical instrument shaft assembly of claim 5, wherein, in the operating position, the proximal outer shaft end (104) extends in a proximal direction (P) from the proximal guide tube end (304).

7. The surgical instrument shaft assembly of claim 5 or 6, wherein the bent section (308) comprises at least one of:
a bend of at least a 90°; and
a circular segment having a radius of one inch or less.

8. The surgical instrument shaft assembly of any of the preceding claims, wherein:
each first segmented floating link (110) has an outer diameter of 0.150 inches or less; and/or
each second segmented floating link (210) has an outer diameter of 0.120 inches or less.

9. The surgical instrument shaft assembly of any of the preceding claims, wherein:
the distal outer shaft end (106) comprises an outer cutter head (118) extending along a first longitudinal axis (122) and having an outer cutter opening (120) in fluid communication with the outer shaft cannula (102) and facing perpendicular to the first longitudinal axis (122); and
the distal inner shaft end (206) comprises an inner cutter head (218) extending along a second longitudinal axis (222) and having an inner cutter opening (220) in fluid communication with the inner shaft cannula (202) and facing perpendicular to the second axis direction;
wherein, in the operating position:
at least a portion of the inner cutter head (218) is surrounded by at least a portion of the outer cutter head (118) with the first longitudinal axis (122) collinear with the second longitudinal axis (222), and
the inner cutter opening (220) is rotatable about the second longitudinal axis (222), upon rotating the inner shaft (200) relative to the outer shaft (100), to selectively move between a first state of alignment between the inner cutter opening (220) and the outer cutter opening (120), and a second state of alignment between the inner cutter opening (220) and the outer cutter opening (120), the second state of alignment being different from the first state of alignment.

10. The surgical instrument shaft assembly of claim 9, wherein, in the first state of alignment, the outer cutter opening (120) and the inner cutter opening (220) form a fluid communication path of a first cross-sectional size with the inner shaft cannula (202), and in the second state of alignment, the outer cutter opening (120) and the inner cutter opening (220) form a fluid communication path of a second cross-sectional size with the inner shaft cannula (202), the second cross-sectional size being less than the first cross-sectional size.

11. The surgical instrument shaft assembly of claim 10, wherein the inner cutter opening (220) is rotatable about the second longitudinal axis (222), upon rotating the inner shaft (200) relative to the outer shaft (100), to selectively move to a third state of alignment between the inner cutter opening (220) and the outer cutter opening (120), and wherein, in the third state of alignment the inner cutter opening (220) and the outer cutter opening (120) do not form a fluid communication path to the inner shaft cannula (202).

12. The surgical instrument shaft assembly of any of the preceding claims, wherein at least one of the inner shaft flexible section (208) and the outer shaft flexible section (108) does not comprise a flexible sheath.

13. A method for operating a surgical instrument according to any of claims 4 through 12, the method comprising:
inserting the inner shaft (200) and the outer shaft (100) into the guide tube (300);
positioning the inner shaft flexible section (208) concentrically within the outer shaft flexible section (108);
positioning the inner shaft flexible section (208) and the outer shaft flexible section (108) to extend throughout the bent section (308);
rotating the outer shaft (100) relative to the guide tube (300); and
rotating the inner shaft (200) relative to the outer shaft (100).

14. The method of claim 13, when dependent upon any of claims 9 through 11, wherein the method further comprises:
positioning the inner cutter head (218) inside the outer cutter head (118) with the first longitudinal axis (122) collinear with the second longitudinal axis (222) and the inner cutter head (218) and outer cutter head (118) at least partially extending in a distal direction (D) from the guide tube (300);
positioning the outer cutter opening (120) adjacent to a region of body tissue (1400);
rotating the inner shaft (200) relative to the outer shaft (100) comprises cyclically moving the inner cutter opening (220) relative to the outer cutter opening (120) to sever a portion of tissue (1402) from the region of body tissue (1400); and
applying suction to the inner shaft cannula (202) to thereby remove the portion of the body tissue (1402) through the inner shaft cannula (202).

15. The method of claim 13 or 14, wherein positioning the outer cutter opening (120) adjacent to the region of body tissue (1400) comprises positioning the guide tube (300) in proximity to the region of body tissue (1400) and rotating the outer shaft (100) relative to the guide tube (300) to orient the outer cutter opening (120) adjacent to the region of body tissue (1400).
